# EUROPEAN PATENT APPLICATION

(11) **EP 4 464 240 A1**
(43) Date of publication of application: **20.11.2024**
(21) Application number: 24176724.3
(22) Date of filing: 17.05.2024
(51) Int. Cl.: A61B 5/00, A61B 5/07

(54) **CAPSULE DEVICE**

(30) Priority: 18.05.2023 TW 112118451
(71) Applicant: Dotspace Inc., Wilmington, DE 19801 (US)
(72) Inventor: JOW, Ueiming, Wilmington, County of New Castle, 19801 (US)
(74) Representative: Patentwerk B.V.

(57) **Abstract**

The capsule device includes a shell having a cavity, a frame disposed in the shell, a circuit board, an antenna module, and a power supply unit. The cavity has a cross section of a first circle. The frame is configured to divide the cavity into at least a first region and a second region. The first region has a cross section at least including a second circle. The center of the second circle deviates from the center of the first circle and away from the second region. The circuit board is arranged in the cavity and includes a senser module. The antenna module is coupled with the circuit board and arranged at the second region. The power supply unit is coupled with the circuit board and arranged at the first region.

## Description

### TECHNICAL FIELD

The present invention relates to a capsule device; in particular, the present invention relates to a capsule device with a frame disposed in the shell of the capsule device.

### BACKGROUND

With the progress of technologies and the rapid development of medical industries, scientists are constantly developing advanced medical equipment or device. For example, in vivo organ electrically stimulating devices or in vivo organ sensors (such as capsule devices) which can be applied to human organs and tissues to assist medical diagnosis or treatment are developed.

Generally, the wireless communication, via antenna, of in vivo sensors are usually limited to the shielding effect of the human body. Also, the signal transmitted by the antenna of the in vivo sensors cannot be completely provided due to absorption of at least a portion of the signal by the human body. The issues for transmitting signals by using the antenna of the in vivo sensors is mainly caused by the complex environment/medium inside the human body. Specifically, an uneven transmission medium may be caused by organs or tissues with different ingredients. Moreover, the capsule devices are in a moving state inside the human body, instead of an immobile state. Therefore, the transmission medium for radiation is unstable, and the impedance of the transmission medium to the antenna is not a fixed value. Accordingly, the transmission frequency of the antenna is prone to frequency deviation due to the difference of the transmission medium, resulting in ineffective transmission.

In addition to the transmission impacts caused by the external environment of the capsule device, the field shape of the antenna of the capsule device is also easily affected by the metal part of the capsule device (such as the battery).

Therefore, how to improve the stability and reliability of signal transmission of capsule devices in a limited inner space will become a major focus of development in this field.

### SUMMARY

One aspect of the present invention is to improve the transmission stability of a capsule device in a complex environment.

Another aspect of the present invention is to reduce the influence of the structure and/or material of the antenna to the antenna field of a capsule device.

The present invention provides a capsule device. The capsule device includes a shell having a cavity, a frame disposed in the shell, a circuit board, an antenna module, and a power supply unit. The cavity has a cross section of a first circle. the frame is configured to divide the cavity into at least a first region and a second region. Wherein the first region has a cross section at least including a second circle. Wherein a center of the second circle deviates from a center of the first circle and away from the second region. The circuit board is arranged in the cavity and comprising a senser module. The antenna module is coupled with the circuit board and arranged at the second region. The power supply unit is coupled with the circuit board and arranged at the first region and inside the frame.

In an embodiment, the frame has a curved wall, a first surface of the curved wall is closely adjacent to the shell, and the second region is formed between the first surface and the shell.

In an embodiment, the frame further has a top plate arranged perpendicular to the curved wall; the top plate further divides the cavity into a third region; the circuit board is arranged at the third region.

In an embodiment, the antenna module is formed on a flexible substrate arranged on the first surface.

In an embodiment, an adhesive layer is arranged between the flexible substrate and the first surface, or the flexible substrate is provided with the adhesive layer.

In an embodiment, a substrate of the antenna module has a first positioning structure; the first surface has a second positioning structure corresponding to the first positioning structure.

In an embodiment, a thickness of the curved wall is equal to or less than a distance from the first surface to an outer surface of the shell.

In an embodiment, the antenna module is formed by a conductor trace arranged on the first surface.

In an embodiment, the conductor trace is arranged on the first surface by laser engraving, embedding, metal stamping, or printing.

In an embodiment, a distance between the center of the first circle and the center of the second circle is equal to or less than a thickness of the shell.

In an embodiment, the antenna module has a plurality of antenna units; each of the plurality of antenna units corresponds to a transmission frequency.

In an embodiment, the circuit board further includes a control/calculate module configured to activate at least one of the plurality of antenna units according to calibration information.

In an embodiment, the antenna module has a signal antenna and a grounding antenna surrounding each other in parallel.

In an embodiment, a distance between the signal antenna and the grounding antenna is less than 3 mm.

In an embodiment, a distance between the signal antenna and the grounding antenna is less than one fourth of a wavelength of a transmitted signal transmitted by the antenna module.

### BRIEF DESCRIPTION OF THE DRAWINGS

The accompanying drawings are presented to help describe various aspects of the present invention. In order to simplify the accompanying drawings and highlight the contents to be presented in the accompanying drawings, conventional structures or elements in the accompanying drawings may be drawn in a simple schematic way or may be omitted. For example, a number of elements may be singular or plural. These accompanying drawings are provided merely to explain these aspects and not to limit them.
FIG. 1 is an exploded view of the components of the capsule device according to the first embodiment of the present invention.
FIG. 2 illustrates a first cross-sectional view of the shell of the capsule device according to the first embodiment of the present invention.
FIG. 3 is a schematic diagram of the internal space of the capsule device according to the first embodiment of the present invention.
FIG. 4 is a schematic diagram of the frame inside the shell according to the first embodiment of the present invention.
FIG. 5 illustrates the first cross-sectional view of the capsule device according to the first embodiment of the present invention.
FIG. 6 is a schematic diagram of the integration of the antenna module and the frame inside the shell according to the first embodiment of the present invention.
FIG. 7 is a second cross-sectional view of the internal space of the capsule device according to the first embodiment of the present invention.
FIG. 8 is a block diagram of the circuit board and the antenna module according to the second embodiment of the present invention.
FIG. 9 is a schematic diagram of the layout of the signal antenna and the grounding antenna according to the third embodiment of the present invention.
FIG. 10 is a simulation diagram of the current density distribution of the signal antenna and the grounding antenna according to the third embodiment of the present invention.
FIG. 11 is a schematic view of the antenna module according to the fourth embodiment of the present invention.
FIGs. 12 and 13 illustrate the simulated radiation fields of the capsule device in a simulated environment according to the fifth embodiment of the present invention.

### DETAILED DESCRIPTION

Any reference to elements using terms such as "first" and "second" herein generally does not limit the number or order of these elements. Conversely, these names are used herein as a convenient way to distinguish two or more elements or element instances. Therefore, it should be understood that the terms "first" and "second" in the request item do not necessarily correspond to the same names in the written description. Furthermore, it should be understood that references to the first element and the second element do not indicate that only two elements can be used or that the first element needs to precede the second element. Open terms such as "include", "comprise", "have", "contain", and the like used herein means including but not limit to.

The term "coupled" is used herein to refer to direct or indirect electrical coupling between two structures. For example, in an example of indirect electrical coupling, one structure may be coupled with another structure through a passive element such as a resistor, a capacitor, or an inductor.

In the present invention, the term such as "exemplary" or "for example" is used to represent "giving an example, instance, or description". Any implementation or aspect described herein as "exemplary" or "for example" is not necessarily to be construed as preferred or advantageous over other aspects of the present invention. The terms "about" and "approximately" as used herein with respect to a specified value or characteristic are intended to represent within a value (for example, 10%) of the specified value or characteristic.

In the present invention, the term "capsule device" refers to a device with a capsule shape. Wherein the capsule device may be configured to perform surgery, delivery or release medicine, sense, and/or operate or control machine. Wherein the capsule device is preferably to arranged in a human body, but not limited to. Wherein the capsule device includes an antenna module configured to coupled communicatively with a host device via a wireless communication. Wherein the wireless communication between the host device and the antenna module of the capsule device can be unidirectional or bidirectional.

### First embodiment:

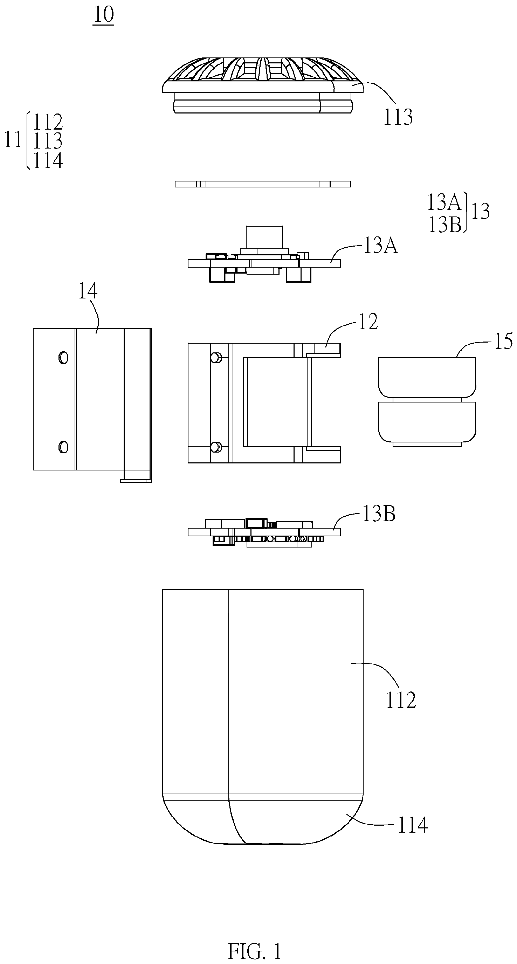

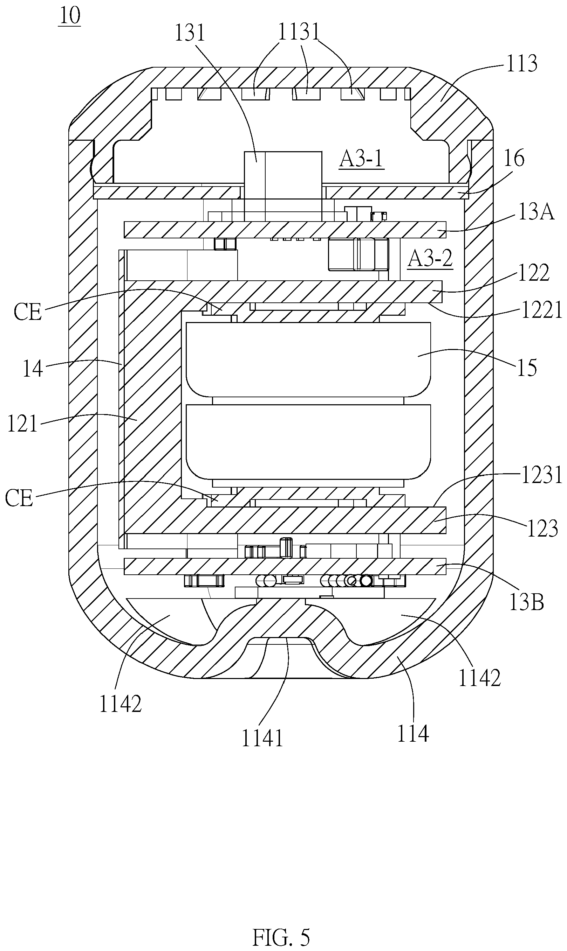

Referring to FIGs. 1 to 7, the capsule device 10 includes a shell 11, a frame 12 disposed in the shell 11, a circuit board 13, an antenna module 14, and a power supply unit 15. The shell 11 has a cavity 111, and the cavity 111 has a cross section of a first circle (C1). The frame 12 is configured to divide the cavity 111 into at least a first region (A1) and a second region (A2). The first region (A1) has a cross section at least including a second circle (C2). The center (C2O) of the second circle (C2) deviates from the center (C1O) of the first circle (C1) and away from the second region (A2). The circuit board 13 is arranged in the cavity 111. The antenna module 14 is coupled with the circuit board 13 and is arranged at the second region (A2). The power supply unit 15 is coupled with the circuit board 13 and is arranged at the first region (A1) and inside the frame 12.

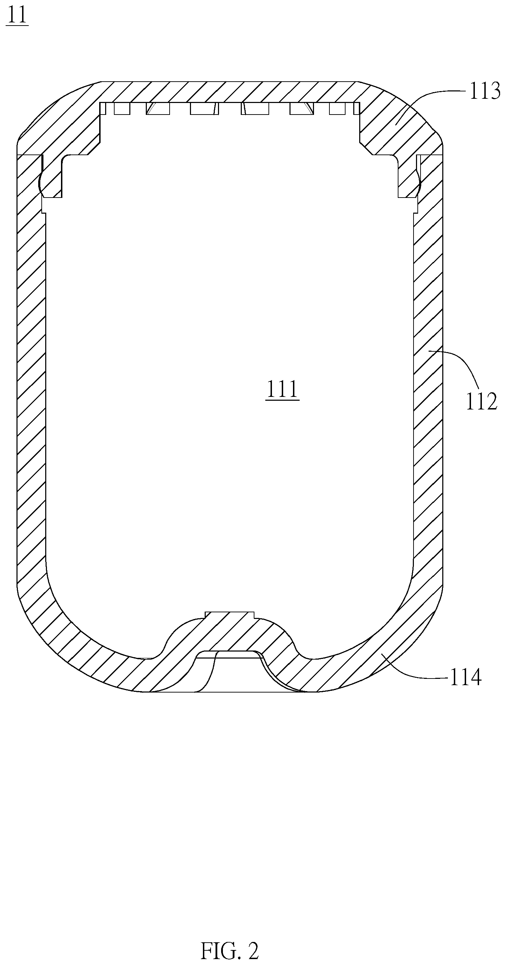

Referring to FIG. 2, FIG. 2 illustrates a first cross-sectional view of the shell 11 along the long axis direction, i.e., the length of the shell. The shell 11 is formed as a capsule shape or the like. Specifically, the shell 11 has a cylindrical wall 112. The shell 11 has an upper cover 113 and a lower dome 114 at two ends of the cylindrical wall 112 to form a capsule shape. The cylindrical wall 112 and the lower dome 114 can be integrally formed to surround the cavity 111. The shell 11 can be made of resin or plastic with better biocompatibility, such as polycarbonate (PC). Alternatively, the shell 11 can be covered by materials with high biocompatibility as a protective layer to protect users from adverse reactions such as toxicity, inflammation, and immunity during the measurement process.

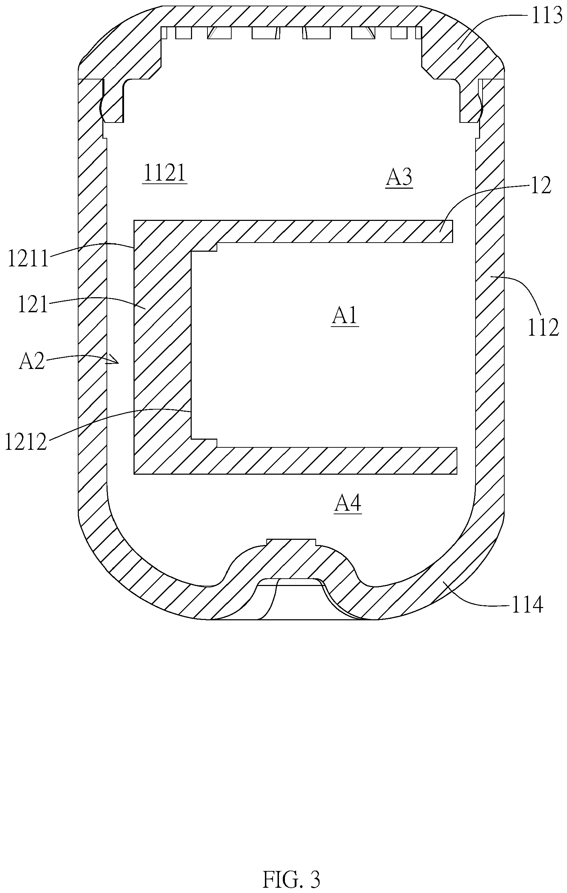

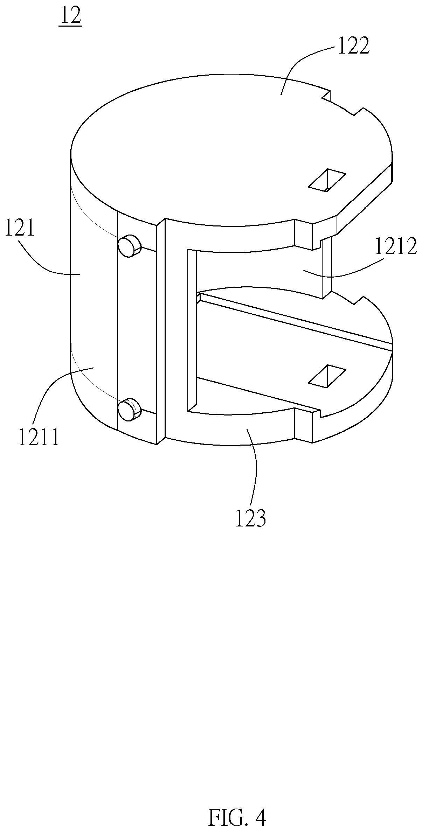

FIG. 3 is the first cross-sectional view of the shell 11 with the frame 12. Referring to FIG. 3, the frame 12 is preferably located in the cylindrical space 1121 (the middle portion of the cavity 111) formed by the cylindrical wall 112. The frame 12 includes a curved wall 121 (as shown in FIG. 4). The first surface 1211 of the curved wall 121 is close to or adjacent to the shell 11 (cylindrical wall 112). The second area (A2) is formed between the first surface 1211 of the curved wall 121 and the shell 11 or the cylindrical wall 112. The gap between the first surface 1211 of the curved wall 121 and the shell 11 is configured to accommodate the antenna module 14. The first area (A1) is formed between the second surface 1212, opposite to the first surface 1211, of the curved wall 121 and the shell 11. It should be noted that the frame 12 inside the shell 11 can be made of materials with higher hardness or rigidity than the shell 11. For example, the material of the frame 12 inside the shell 11 can be polyamide (PA). Therefore, the material of the shell 11 provides the function of better biocompatibility, and the material of the frame 12 provides support and rigidity to increase the durability of the capsule device 10. Accordingly, the frame 12 prevents damage to the capsule device 10 due to pressure inside the subject's body or force exerted by oral bite.

In the embodiment, referring to FIGs. 3 to 5. The frame 12 inside the shell 11 may also have an upper plate 122 and a lower plate 123 arranged vertically with the curved wall 121. The lower plate 123 of the frame 12 is preferably disposed at the junction of the lower dome 114 and the cylindrical space 1121. A third region (A3) is formed between the upper plate 122 of the frame 12 and the upper cover 113 of the shell 11. A fourth space (A4) is formed between the lower plate 123 and the lower dome 114. The circuit board 13 is arranged in the third region (A3) and/or the fourth space (A4). For example, the circuit board 13 can be divided into an upper circuit board (13A) and a lower circuit board (13B) according to the circuit layout or individual modules used in the circuit board 13. The upper circuit board (13A) is arranged in the third region (A3) and lower circuit board (13B) is arranged in the fourth space (A4). It should be noted that the first space (A1), the second space (A2), the third region (A3), and/or the fourth space (A4) may not necessarily be an enclosed space. For example, the frame 12 can completely isolate the cavity 111 to enclose the first space (A1), the second space (A2), the third region (A3), and/or the fourth space (A4). Alternatively, the frame 12 can roughly partition the cavity 111 in the shell 11 to form the first space (A1), the second space (A2), the third region (A3), and/or the fourth space (A4) instead of completely isolating the cavity 111.

In an embodiment, the circuit board 13 may be a printed circuit board. In an embodiment, the circuit board 13 may include an operation module 131, e.g. a sensor. The operation module 131 is configured to perform surgery, delivery or release medicine, sense, and/or operate or control machine, but not limited to. In an embodiment, the operation module 131 is preferably a sensor such as pressure, pH, temperature, drug concentration, gas concentration, or optical sensing, but not limited thereto. After the capsule device 10 enters the subject's body, the operation module 131 is controlled or communicated with a host device. For example, the operation module 131 is a sensor for measuring and creating sensing signal(s). The sensing signal(s) is provided to the host device through a wireless communication by the antenna module 14. The host device can be any possible device with communication capabilities such as a computer, a wearable device, or a smartphone, but not limited thereto.

The power supply unit 15 is arranged in the first space (A1) and inside the frame 12. For example, the power supply unit 15 is a battery and preferably one or more series connected button batteries or miniature batteries. The surface 1221 of the top plate 122 facing the first space and the surface 1231 of the bottom plate 123 facing the first space can be equipped with contact conductors (CE) as the electrodes of the power supply unit 15. The power supply unit 15 is coupled to the circuit board 13 through the contact conductors (CE). As such, the power supply unit 15 can supply power to the circuit board 13 via the contact conductors (CE).

A sealing layer 16 can be disposed in the third region (A3) and configured to separate the third region (A3) into an upper half portion (A3-1) and a lower half portion (A3-2). The upper half portion (A3-1) is a semi-open space to allow gas or liquid to flow in/out the upper half portion (A3-1) through the opening 1131 of the upper cover 113. The lower half portion (A3-2) can accommodate the upper circuit board (13A) with the operation module 131. The operating part (such as for sensing) of the operation module 131 may extend into the upper half portion (A3-1) through the sealing hole formed on the sealing layer 16, and preferably at the center of the sealing layer 16. The sealing layer 16 will prevent gas or liquid from the measuring environment from flowing into the lower half portion (A3-2) or other spaces (such as space A1, A2, or A4).

In another aspect, the center 1141 of the lower dome 114 can be recessed toward the fourth space (A4) to provide a better support for internal parts (such as the frame 12 and/or the circuit board 13), but the reason for setting the recessed lower dome 114 is not limited to this. In addition, the center 1141 of the lower dome 114 can be equipped with ribs or support walls 1142 to increase the structural strength. Therefore, the lower dome 114 with such a structure enhanced feature will improve the stability of the shell 11 or other components inside the capsule device 10.

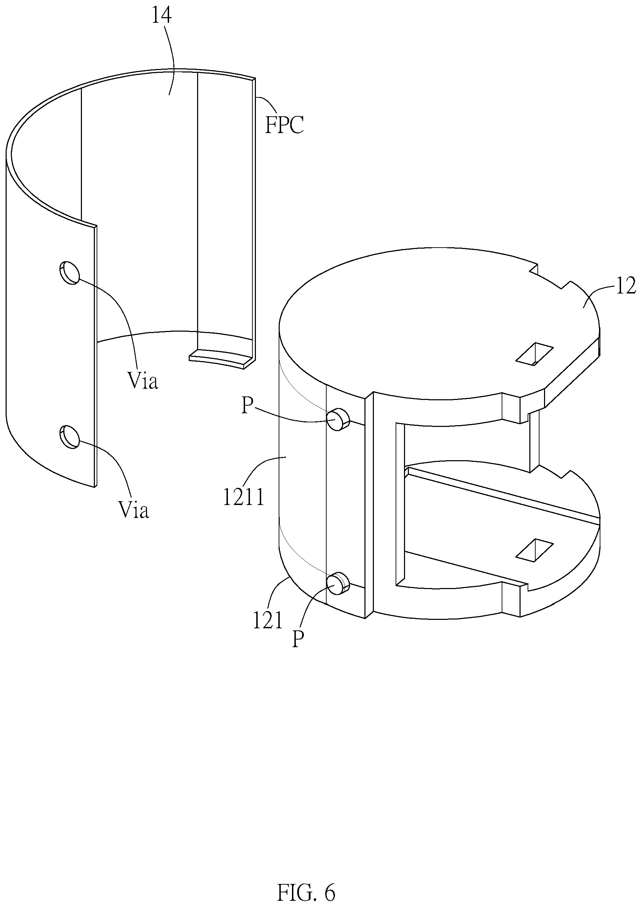

Referring to FIG. 6, the antenna module 14 arranged in the second space (A2) can be made conductor traces formed on a flexible circuit board (FPC), and the FPC is disposed on the first surface 1211 of the curved wall 121 by positioning structures on the FPC and the first surface 1211. For example, a first positioning structure (such as via) can be disposed on the FPC, and a second positioning structure (e.g. positioning pilar (p)) is disposed on the first surface 1211 corresponding to the first positioning structure. When the FPC is disposed on the first surface 1211, the alignment and installation can be conveniently achieved by combining the first positioning structure and the second positioning structure to ensure the consistency in the product manufacturing process and prevent poor installation that may affect the efficiency and field shape of the antenna module 14. In the embodiment, the antenna module 14 can be fixed through an adhesive layer. For example, the adhesive layer is disposed between the antenna module 14 and the first surface 1211 to fix the antenna module 14 on the first surface 1211. In another embodiment, the antenna module 14 can be arranged by tape or double-sided tape on the first surface 1211, or the FPC is provided with the adhesive layer. The tape or double-sided tape can wrap around the antenna module 14 to fix the antenna module 14 on the first surface 1211.

The antenna module 14 can be formed by laying out conductor traces on the first surface 1211. For example, a conductor layer can be disposed on the first surface 1211 of the curved wall 121, and the conductor traces can be formed by removing a part of the conductor layer by mechanical carving, laser carving, etching, or other means, but not limited thereto. The conductor traces can be formed by any conventional processing methods, such as embedding or printing. In addition, the antenna module 14 can be made into metal sheets through metal stamping and placed on the first surface 1211 of the curved wall 121. Due to the sheet-like configuration of antenna module 14, the space required for accommodating the antenna module 14 (e.g. second area A2) can be reduced. Also, the use of mechanically processed conductor traces or metal sheets can maintain the consistency of the antenna module 14, avoiding poor installation that affects the efficiency and field shape of the antenna.

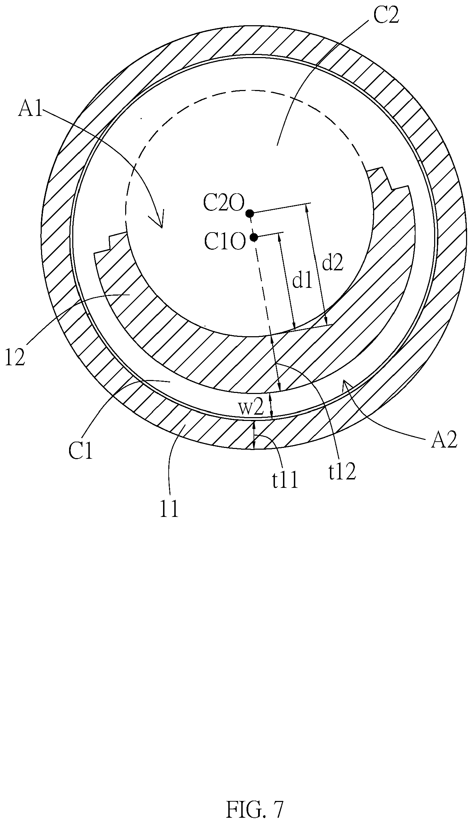

In the present invention, in order to reduce the influence of power supply unit 15 on the field shape of the antenna module 14, the power supply unit 15 and the antenna module 14 are preferably separated by the frame 12. The power supply unit 15 is preferably disposed as far away from the antenna module 14 as possible. More specifically, FIG. 7 illustrates a second cross-sectional view of the capsule device 10 along a direction parallel to the radial direction of the capsule device 10. The second cross section of the first region (A1) contains the second circle (C2), wherein the second circle (C2) is roughly equivalent to the cross section of the power supply unit 15. In other words, the power supply unit 15 is coaxial with the center (C2O) of the second circle (C2). Moreover, the center (C2O) of the second circle (C2) deviates from the center (C1O) of the first circle (C1) and is relatively away from the second region (A2). Specifically, the distance (d2) from the center (C2O) of the second circle (C2) to the curved wall 121 will be greater than the distance (d1) from the center (C1O) of the first circle (C1) to the curved wall 121. In the cavity 111, the second area (A2) for accommodating the antenna module 14 and the first area (A1) for accommodating the power supply unit 15 are separated by the frame 12 to prevent the power supply unit 15 from influencing the field shape of the antenna module 14. Preferably, the distance between the center (C1O) of the first circle (C1) and the center (C2O) of the second circle (C2) is half or less than the thickness (t11) of the shell 11 (i.e. d2-d1 ≤ t11). Accordingly, the allocation of the first region (A1) and/or the second region (A2) within the cavity 111 of the shell 11 is more effectively to provide a good antenna signal transmission environment without affecting the installation of power supply unit 15.

In an embodiment, the thickness (t12) of the curved wall 121 of the frame 12 is equal to or less than the sum of the width (w2) of the second region (A2) and the thickness (t11) of the shell 11 (i.e., t12 ≤ w2+t11). Preferably, the thickness (t12) of the curved wall 121 is about 1.2-1.5 mm, and the thickness (t11) of the shell 11 is about 0.7-1.0 mm. In the embodiment, the distance between the antenna module 14 and the power supply unit 15 is equal to or less than the distance between the antenna module 14 and the outer surface of the shell 11 (such as the outer surface of the cylindrical wall 112). Specifically, the organisms or tissues that will contact with the outer surface of shell 11 can be treated as conductors. By properly configuring the distance between the antenna module 14 and the conductors (such as power supply unit 15 inside the shell 11, biological organisms outside the shell 11), the transmission efficiency of the antenna module 14 can be effectively improved, and the transmission field can be optimized. However, the method of adjusting the distance between antenna module 14 and the conductor is not limited to this embodiment.

Second embodiment:

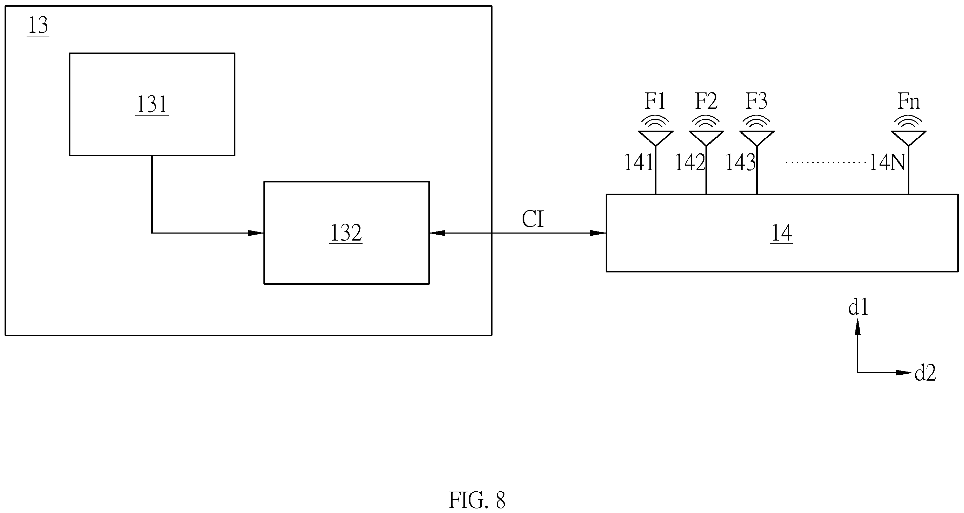

As shown in FIG. 8, the antenna module 14 includes multiple antenna units (141-14N). Each of the antenna units (141-14N) is configured to transmit signals corresponding to a respective transmission frequency from frequences (F1-FN). When the capsule device 10 is located within a complicated environment (such as complex medium), the complicated environment usually results in poor transmission of the signal from the antenna module 14 or poor reception of the signal by a remote device due to frequency deviation. Therefore, one or more optimal antenna units with an appropriate frequency corresponding to the complex medium can be selected or activated from the antenna units (141-14N). The antenna module 14 including the antenna units (141-14N) can provide a better transmission frequency for the capsule device 10.

In the embodiment for configuring the antenna units (141-14N), the circuit board 13 may further include a control/calculation module 132, which is configured to activate at least one of the antenna units (141-14N) based on the calibration information (CI). More specifically, the calibration information (CI) may be provided by the remote device or the control/calculation module 132 to activate and/or deactivate at least one of antenna units (141-14N) according to the transmission performance of the working antenna unit(s) of antenna units (141-14N). The calibration information (CI) may be determined by the transmission performance of the least one of antenna units (141-14N), such as signal strength, signal vector, and/or other signal parameters. However, the activation/deactivation of the antenna units (141-14N) is not limited to the above embodiment. For example, the control/calculation module 132 can select the optimal antenna unit(s) for signal transmission by scanning or polling the antenna units (141-14N) for optimal parameters, but not limited thereto.

### Third embodiment:

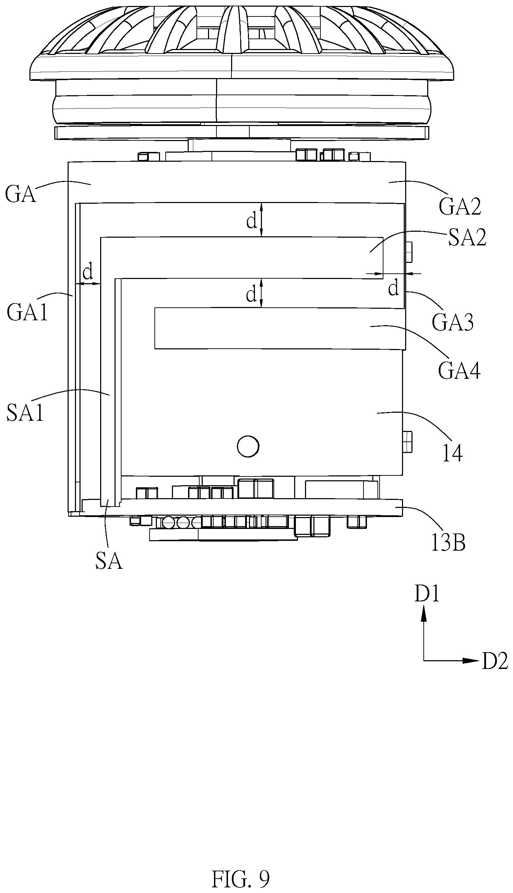

FIG. 9 illustrates the antenna module 14 having a signal antenna (SA) and a grounding antenna (GA) arranged parallel to the signal antenna (SA). Specifically, the signal antenna (SA) is an L-shaped conductor with a first signal arm (SA1) and a second signal arm (SA2). One end of the first signal arm (SA1) is coupled to the circuit board 13 (e.g. the lower circuit board (13B), but not limited thereto). The other end of the first signal arm (SA1) is connected to the second signal arm (SA2). The second signal arm (SA2) extends in a direction (D2) perpendicular to an extension direction (D1) of the first signal arm (SA1) and is preferably longer than the first signal arm (SA1). On the other hand, the grounding antenna (GA) has a first grounding arm (GA1), a second grounding arm (GA2), a third grounding arm (GA3), and a fourth grounding arm (GA4). Similar to the signal antenna (SA), one end of the first grounding arm (GA1) is coupled to the circuit board 13, and the other end is coupled to the second grounding arm (GA2). The third grounding arm (GA3) and the fourth grounding arm (GA4) are sequentially connected at right angle. The first signal arm (SA1) and the first grounding arm (GA1) extend parallel to each other. The second signal arm (SA2) and the second grounding arm (GA2) extend parallel to each other. The third grounding arm (GA3) and the fourth grounding arm (GA4) form an L-shape and surround the second signal arm (SA2). In this embodiment, the gap or distance (d) between the signal antenna (SA) and the grounding antenna (GA) is preferably less than 3 mm or one fourth of the wavelength ( *λ* ) of the transmitted signal (i.e., d < λ/10).

It should be noted that the layout of the conducting portion of the antenna module 14 is not limited to FIG. 9. Specifically, the direction (D1) and the direction (D2) can be adjusted based on the arc length and the width of the curved wall 121 (the distance between the upper plate 122 and the lower plate 123). For example, when the arc length is shorter than the width of the curved wall 121 (i.e., the capsule shape is thick and short), the first grounding arm (GA1) may firstly extend along the direction of the arc length, and the second grounding arm (GA2) then extends along the wall surface of the curved wall 121. When the arc length is longer than the width of the curved wall 121 (i.e., the capsule shape is slender), the first grounding arm (GA1) may firstly extend along the wall surface of the curved wall 121, and the second grounding arm (GA2) then extends along the direction of the arc length. In other words, the layout of the antenna module 14 can be adjusted according to the shape of the shell 11.

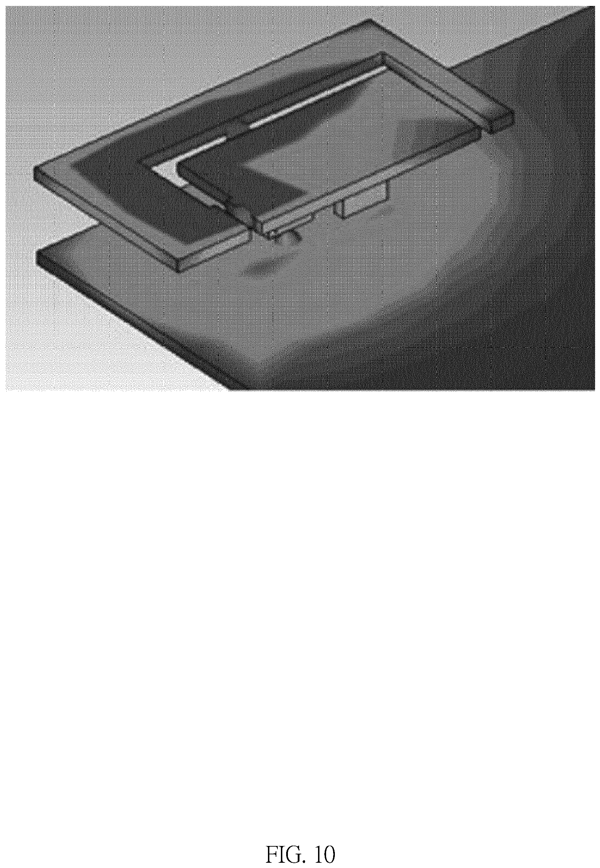

In this embodiment, the signal antenna (SA) will couple the grounding antenna (GA). The coupling effect between the signal antenna (SA) and the grounding antenna (GA) will provide at least one resonance frequency. For example, the antenna module 14 has a first resonance frequency (e.g. 2.5 GHz) and a second resonance frequency (e.g. 3.5 GHz). It should be noted that the first resonance frequency and/or the second resonance frequency of the present invention are not limited to the above frequencies. The resonance frequency of the antenna module 14 can be adjusted by modifying the signal antenna (SA) and the grounding antenna (GA) through any means (for example, modifying the distance between the signal antenna (SA) and the grounding antenna (GA) or other conventional modifications). The signal antenna (SA) and the grounding antenna (GA) are modified to cause the first resonance frequency close to the second resonance frequency. The medication of first resonance frequency close to the second resonance frequency widens the bandwidth of the antenna module 14. Due to the widening of the bandwidth of the antenna module 14, the impact of frequency deviation caused by the complex medium for setting the capsule device 10 will be reduced. In addition, by separating the antenna module 14 into two antenna parts, the energy shock to the object will be reduced compared to a single antenna design. More specifically, the energy of the electromagnetic waves provided by the single antenna design is concentrated to a single point. Therefore, the energy shock of the electromagnetic wave per unit area of the object will increase, and the radiation effect provided to the object will also increase. A simulation of the energy intensity of the antenna module 14 with two antenna parts is illustrated in FIG. 10. As shown in FIG. 10, the antenna module 14 has the signal antenna (SA) and the grounding antenna (GA) formed as a bipolar arm. The current of the signal antenna (SA) is coupled to the grounding antenna (GA). Therefore, the near-field current is dispersed. The dispersed near-field current also disperses the extreme intensity of near-field radiation. Accordingly, the bipolar arm design of the antenna module 14 reduces the electromagnetic wave density of the antenna module 14 and the radiation risk to the object.

### Fourth embodiment:

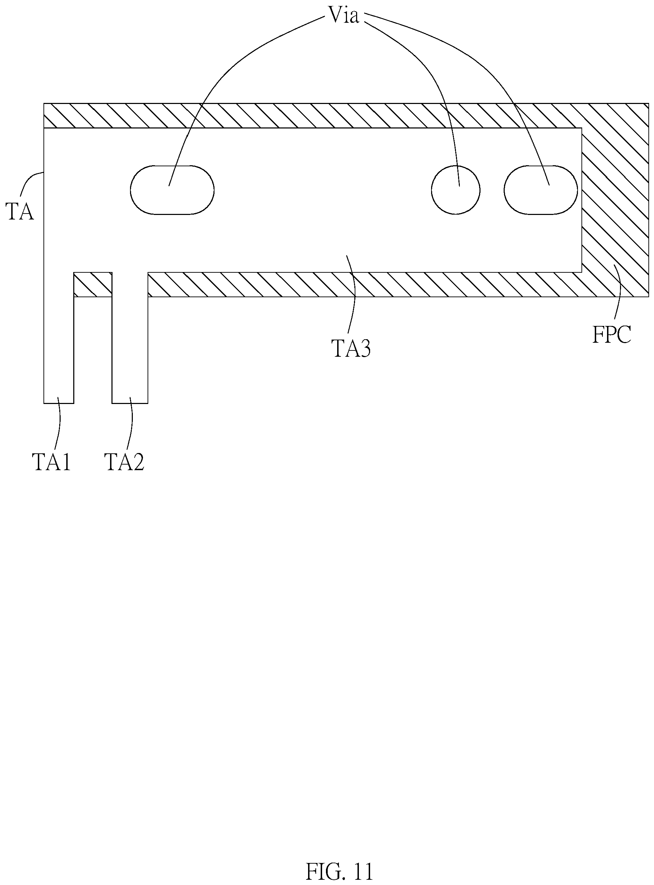

In the embodiment of the antenna module 14 shown in FIG. 11, the antenna module 14 includes a flexible substrate (FPC) and a conductor trace (TA) disposed on the flexible substrate (FPC). The conductor trace (TA) has two contacts (TA1, TA2) circuit board and a radiation part (TA3), which can be integrally formed. The contacts (TA1, TA2) are coupled to the circuit board 13. The antenna module 14 with the flexible substrate (FPC) can be made by any conventional process for manufacturing circuit on a substrate. Through a mature manufacturing process, the difficulty of aligning the antenna module 14 can be reduced, and the yield for producing the antenna module 14 can be improved. In the embodiment, the radiation part (TA3) and/or the flexible substrate (FPC) can be combined by with a first positioning structure (such as a perforated via) to improve the installation convenience and fix the antenna. The first positioning structure will prevent failure of transmission/reception caused by poor installation. It should be noted that the present invention is not limited to the shape and size of the radiation part (TA3) shown in FIG. 11. The shape and size of the radiation part (TA3) can be modified according to any means for designing antenna in the technical field.

### Fifth embodiment:

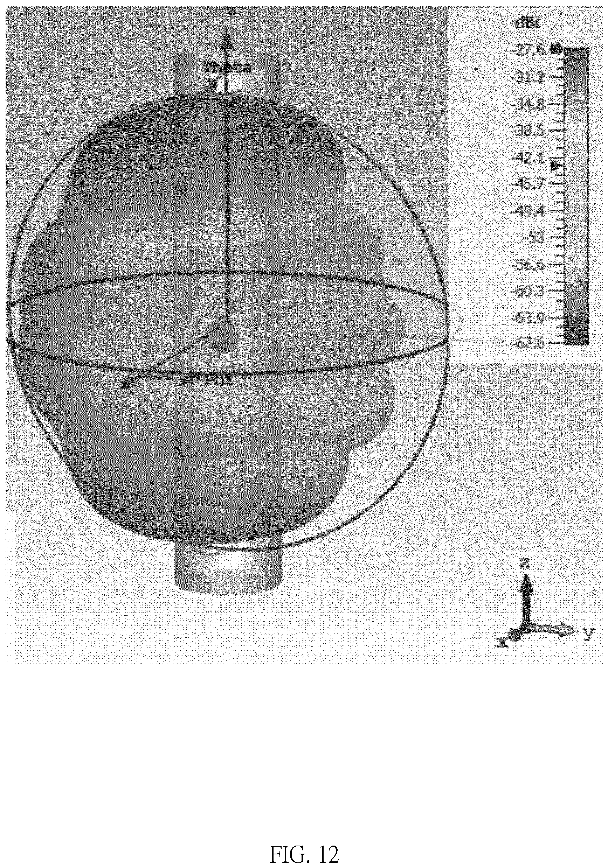

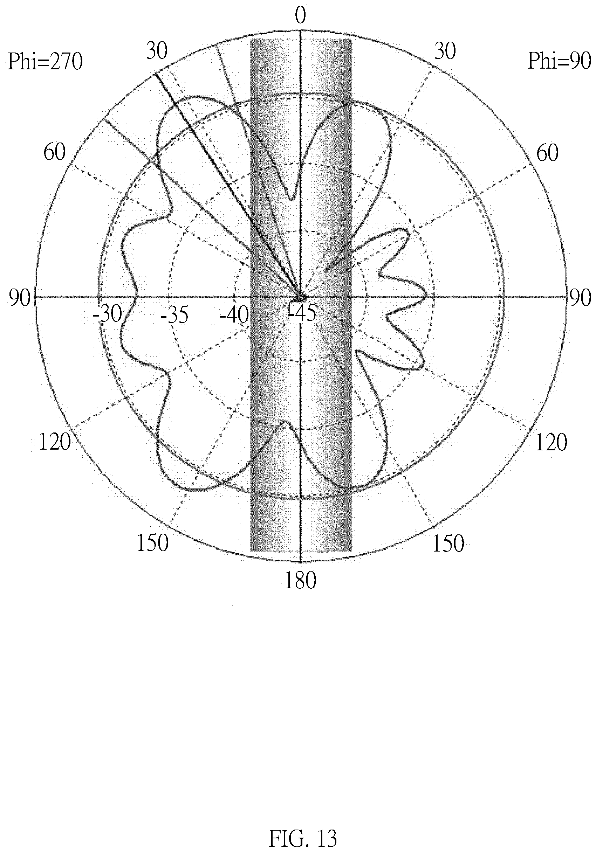

FIGs. 12 and 13 illustrate the simulated radiation field generated by the capsule device of the present invention in a biomimetic environment (such as saline water). The capsule device of the present invention has the frame and the antenna module corresponding to the frame. The frame arranged inside the shell prevents the radiation emitted by the antenna module from being affected by the internal structure of the capsule device (e.g. the battery or other metal or conductive components inside the capsule device). Referring FIGs. 12 and 13, the antenna module has a good radiation field emitted toward outside of the shell. The capsule device of the present invention effectively configures the second area for the antenna module and the first area for the power supply unit in a limited space through the frame inside the shell. The frame divides the inner space of the capsule device with a shifting central axis to the power supply unit. Accordingly, the frame reduces the influence of the radiation field of the antenna module, which is affected by the power supply unit or other conducting components in the capsule device. Therefore, the capsule device will achieve an improved transmission stability.

The previous description of the present invention is provided to enable a person of ordinary skill in the art to make or implement the present invention. Various modifications to the present invention will be apparent to a person skilled in the art, and the general principles defined herein can be applied to other variations without departing from the spirit or scope of the present invention. Therefore, the present invention is not intended to be limited to the examples described herein, but is to be in accord with the widest scope consistent with the principles and novel features of the invention herein.

## Claims

1. A capsule device, comprising:
a shell having a cavity, the cavity having a cross section of a first circle;
a frame disposed in the shell and configured to divide the cavity into at least a first region and a second region, the first region having a cross section at least including a second circle, a center of the second circle deviating from a center of the first circle and away from the second region;
a circuit board arranged in the cavity;
an antenna module coupled with the circuit board and arranged at the second region; and
a power supply unit coupled with the circuit board and arranged at the first region and inside the frame.

2. The capsule device of claim 1, wherein the frame has a curved wall, a first surface of the curved wall is closely adjacent to the shell, and the second region is formed between the first surface and the shell.

3. The capsule device of claim 2, wherein the frame further has a top plate arranged perpendicular to the curved wall; the top plate further divides the cavity with a third region; the circuit board is arranged at the third region.

4. The capsule device of claim 2, wherein the antenna module is formed on a flexible substrate arranged on the first surface.

5. The capsule device of claim 4, wherein an adhesive layer is arranged between the flexible substrate and the first surface, or the flexible substrate is provided with the adhesive layer.

6. The capsule device of claim 4, wherein a substrate of the antenna module has a first positioning structure; the first surface has a second positioning structure corresponding to the first positioning structure.

7. The capsule device of claim 4, wherein a thickness of the curved wall is equal to or less than a distance from the first surface to an outer surface of the shell.

8. The capsule device of claim 2, wherein the antenna module is formed by a conductor trace arranged on the first surface.

9. The capsule device of claim 8, wherein the conductor trace is arranged on the first surface by laser engraving, embedding, metal stamping, or printing.

10. The capsule device of claim 1, further comprises an operation module coupled to the circuit board; wherein the operation module is configured to perform surgery, delivery or release medicine, sense, and/or operate or control machine.

11. The capsule device of claim 1, wherein a distance between the center of the first circle and the center of the second circle is equal to or less than a thickness of the shell.

12. The capsule device of claim 1, wherein the antenna module has a plurality of antenna units; each of the plurality of antenna units corresponds to a transmission frequency.

13. The capsule device of claim 12, wherein the circuit board further includes a control/calculation module configured to activate at least one of the plurality of antenna units according to calibration information.

14. The capsule device of claim 1, wherein the antenna module has a signal antenna and a grounding antenna surrounding each other in parallel.

15. The capsule device of claim 14, wherein a distance between the signal antenna and the grounding antenna is less than 3 mm.

16. The capsule device of claim 14, wherein a distance between the signal antenna and the grounding antenna is less than one fourth of a wavelength of a transmitted signal transmitted by the antenna module.
